# EUROPEAN PATENT SPECIFICATION

(11) **EP 0 531 401 B1**
(45) Date of publication and mention of the grant of the patent: **22.01.1997**
(21) Application number: 91910429.9
(22) Date of filing: 03.05.1991
(51) Int. Cl.: A61K 39/00, A61K 38/00, A61K 49/00, A61K 39/39

(54) **ENHANCING THE ASSOCIATION OF EXOGENOUS PEPTIDES WITH CLASS I MHC MOLECULES ON IMMUNE SYSTEM CELLS**
ERHÖHUNG DER ASSOZIATION EXOGENER PEPTIDE MIT MHCI-MOLEKÜLEN AUF ZELLEN DES IMMUNSYSTEMS
RENFORCEMENT DE L'ASSOCIATION DE PEPTIDES EXOGENES AVEC DES MOLECULES MHC DE LA CLASSE I SUR DES CELLULES DE SYSTEMS IMMUNS

(30) Priority: 10.05.1990 US 521576
(43) Date of publication of application: 17.03.1993
(73) Proprietor: DANA FARBER CANCER INSTITUTE, Boston Massachusetts 02115 (US)
(72) Inventor: ROCK, Kenneth, L., West Roxbury, MA 02132 (US)
(74) Representative: Wössner, Gottfried
(86) International application number: US9103082
(87) International publication number: WO9116924

(56) References cited:
- WO-A-79/00160
- WO-A-80/01986
- NATURE, vol. 308, 12 April 1984; BERNABEU et al., pp. 642-645
- SCIENCE, vol. 239, 05 February 1988; YEWDELL et al., pp. 637-640
- PROCEEDINGS OF THE NATL. ACADEMY OF SCIENCES USA, vol. 76, no. 11, November 1979, Washington, DC (US); HYATIL et al.,pp. 5834-5838
- CELL, vol. 54, 09 September 1988; MOORE et al., pp. 777-785
- JOURNAL OF EXPERIMENTAL MEDICINE, vol. 167, June 1988; CARBONE et al., pp. 1767-1779

## Description

### Technical Field of Invention

The present invention relates to a method for enhancing the association of exogenous peptides with class I MHC molecules on the surface of antigen presenting cells of the immune system and to the extension of this methodology to vaccination with synthetic peptides to prime for cytotoxic T lymphocyte response.

### Background of the Invention

Vaccines have long been utilized by man to aid in the prevention of infectious diseases in both humans and animals. Active immunization procedures have more recently been tested in other areas, such as the prevention and treatment of malignant disorders. The use of vaccines is based upon the stimulation of the specific immune response, which includes both humoral and cellular components. Although vaccination is often considered in terms of its ability to stimulate antigen-specific antibody, vaccine effectiveness is not necessarily determined by the induction of serum antibody alone (humoral factors). Rather it is determined by the demonstration of enhanced protection against disease, which may include cytotoxic T lymphocyte response. Cytotoxic T lymphocytes provide an effective defense against many intracellular pathogens. In some cases, full protection can be achieved even in the absence of an antibody response.

Vaccines have historically been prepared by killing or attenuating pathogenic organisms, e.g. viruses, and then injecting the resulting virus particles into a patient or host mammal. One of the major problems with these vaccines is that they always involve an inherent threat that the virus may not be sufficiently attenuated or killed. There is thus the potential for the vaccine itself to cause the disease against which protection is sought. Another limitation of this approach is that most pathogens, when killed, do not effectively stimulate cytotoxic T lymphocyte immunity.

The threat of unattenuated pathogens can sometimes be overcome by using a specific component of the pathogen. The component used is typically a protein from, for example, a viral capsid or envelope which forms an outer portion of the virus. As with the attenuated vaccines however, this approach is also limited in that most protein antigens which are introduced into the extracellular fluid do not effectively stimulate cytotoxic T lymphocyte immunity. In addition, this method also poses the possibility of a pathogenic response. A still further limitation is that the thus-produced vaccine may include antigens that compete with or are detrimental to the immune response and may cause undesirable side effects.

More recently, certain pathogen-related proteins have been immunologically mimicked with synthetic polypeptides whose amino acid sequence corresponds to that of an antigenic determinant domain of the pathogen-related protein. For example, U.S. Patent No. 4,794,168 describes leukemia associated immunogens that are relatively short peptide sequences corresponding to the antigenic determinant domains of a leukemia associated virus envelope protein. Similarly, other peptide immunogens have been reported by Sutcliffe et al, Nature, Vol. 287, pp. 801-805 (1980); Lerner et al, Proc. Natl. Acad. Sci. U.S.A., Vol. 78, pp. 347-47 (1981) and Bittle et al, Nature, Vol. 298, pp. 30-33 (1982). The peptide sequence of a natural protein can be determined either from the protein itself or from the nucleotide sequence of the genome encoding the protein.

Although peptide immunization can stimulate class II MHC-restricted T lymphocytes to help B lymphocytes secrete antibody (helper T cells) in a host, induction of a CTL response usually requires in vivo priming with infectious virus. Most studies have indicated that class I MHC cytotoxic T lymphocytes are rarely, if ever, induced with peptide immunizations. See, Bevan, "Stimulating Killer T Cells", Nature, Vol. 342, pp. 478-79 (November 1989). The general inability of synthetic polypeptides to prime cytotoxic T lymphocytes in vivo thus presents a formidable hurdle to the use of such peptides in effective vaccination.

There have been isolated reports in the literature of peptides which can prime CTL responses in vivo. For example, Deres et al recently reported that synthetic viral peptides covalently linked to tripalmitoyl-S-glycerylcysteinyl-seryl-serine can effectively prime influenza virus specific CTL in vivo. Nature, Vol. 342, pp. 561-62 (November 1989). Carbone and Bevan described in vivo priming of CTL with partial tryptic digests of ovalbumin (OVA) and a specific OVA peptide, OVA₂₂₉₋₂₇₆, although all other OVA peptides tested, including peptides from a complete tryptic digest of OVA, were unable to prime CTL's. J. Exp. Med., Vol. 169, pp. 603-11 (March 1989). The most often provided explanation for the ability of a few peptides to serve as in vivo immunogens for CTL is the ability of the peptides to enter into the cytoplasm of antigen-presenting cells, where they can enter the normal pathway that leads to association with class I molecules of the MHC.

By way of further background, cytotoxic T lymphocytes and T helper cells generally recognize processed antigenic peptides in association with class I and class II major histocompatibility (MHC) gene products, respectively, on the surface of antigen presenting cells (APC's). It is currently believed by those skilled in the art that the processed peptides arise from cleavage of protein antigens either in the cytosolic/endoplasmic reticulum or endosomal compartments of cells. Class I molecules are expressed on all cells and are meant to display a sample of internally synthesized peptides by the cells for surveillance by cytolytic T cells. It is generally believed that the cytosolic peptides which arise from antigens that are synthesized by cells bind to newly synthesized MHC molecules.

In contrast to the class I molecules, class II molecules have a limited expression in cells concerned with the presentation of foreign protein antigens to initiate the immune response. Antigens from the extracellular fluids are processed in endosomes and subsequently bind to class II molecules. Antigens that are acquired from the extracellular fluid are thus presented in association with class II molecules, while those that are synthesized endogenously are presented in association with class I molecules. Germain, Nature, Vol. 332, pp. 687-89 (1986); Braciale et al., Immunol. Rev., pp. 95-114 (1987). A general inability of most antigens to enter into the class I antigen presentation pathway from extracellular fluid could thus account for the inability of such antigens to prime in vivo despite the achievement of effective priming of T helper cells, which are class II MHC-restricted. The reason for the general inability of synthetic peptides to prime class I MHC restricted T cells in vivo is not known.

As intimated above, the form in which antigen interacts with and is displayed by both class I and class II MHC is thought to be as a small peptide. If this is indeed the case, it would be a logical expectation that small peptides might bind to class I MHC molecules on the cell surface and thus bypass the intracellular processes and be presented to the immune system, when the antigen is presented in the form of small peptides in the extracellular fluid. This apparently does occur in vitro. For example, Maryanski et al demonstrated that certain peptides can sensitize class I MHC antigen presenting cells for lytic action by antigen specific cytotoxic T lymphocytes at high doses of exogenous peptides in vitro. Nature, Vol, 324, No. 11 (1986). See also, Townsend et al, Cell, Vol. 44, pp. 959-68 (March 1986).

Peptide fragments at high concentrations can also prime naive populations of lymphocytes for cytotoxic T cell response in culture. Carbone et al, J. Exp. Med., Vol. 167, pp. 1767-79 (June 1988). Experiments with fixed antigen presenting cells have shown that efficient access to the endogenous processing pathway of class I MHC antigen presenting cells is not required for antigen presentation to CTL in vitro under these culture conditions. Although it is not clear why in vitro priming has been successful, there is some suggestion that the high concentrations of peptides typically employed in in vitro cultures may allow intracellular complexes to form or may drive the association of peptides directly with unoccupied class I molecules on the cell surface. Use of high peptide concentrations in vitro may be sufficient for study of the events of target cell sensitization and priming for CTL response. Such culture conditions may not, however, be the most efficent conditions with which to cause association of polypeptides and/or synthetic peptides with class I MHC molecules.

Despite the observations regarding in vitro sensitization and priming for CTL response, the events generally do not occur in vivo in a manner that has allowed stimulation of cytotoxic T lymphocyte response.

One possible explanation for this is that the concentration of circulating peptides is not maintained at a high enough level for this type of presentation to be effective in vivo. Even if sufficiently high concentrations of synthetic peptide could be maintained in a host organism to initiate CTL response, the cost of using such high concentrations of peptides with present technology would be prohibitive. Synthetic peptides are extremely expensive and vaccines requiring high concentrations of peptide would be unattractive for these reasons.

Accordingly, it is an object of the present invention to provide a method for enhancing the association of exogenous polypeptides with class I MHC-restricted molecules on the surface of antigen presenting cells.

Another object of the present invention is to provide a method for priming class I MHC-restricted cytotoxic T cell response with polypeptides.

Yet another object of the present invention is to provide a method for priming cytotoxic T cell response in vivo with polypeptides.

A still further object of the present invention is to provide an adjuvant that can be utilized with synthetic peptide vaccines to enhance the immunogenicity of pathogen-related peptides.

### Summary of the Invention

These as well as other objects and advantages are accomplished by the present invention, which provides a method for enhancing the association of exogenously prepared polypeptides with class I MHC molecules on the surface of antigen presenting cells to thereby sensitize the target cells for class I MHC-restricted cytotoxic T cell response. The method of the present invention involves obtaining the polypeptides of interest and presenting the polypeptides to the appropriate antigen presenting cells in the presence of an elevated level of free β₂-microglobulin. The concentration of β₂-microglobulin employed in the method of the present invention is elevated to a level higher than that normally present as free β₂-microglobulin in mammalian serum.

The method of the present invention can also be used to prime naive populations of T lymphocytes for antigen-specific class I MHC-restricted cytotoxic T cell response with exogenous polypeptide and is useful in both in vitro and in vivo applications. In accordance with the present invention, a new use for purified β₂-microglobulin as an adjuvant for synthetic polypeptide vaccines is also provided. In vivo immunization with the synthetic peptides and free β₂-microglobulin can elicit an antigen-specific response as strong as that produced with endogenously synthesized antigen.

### Brief Description of the Drawings

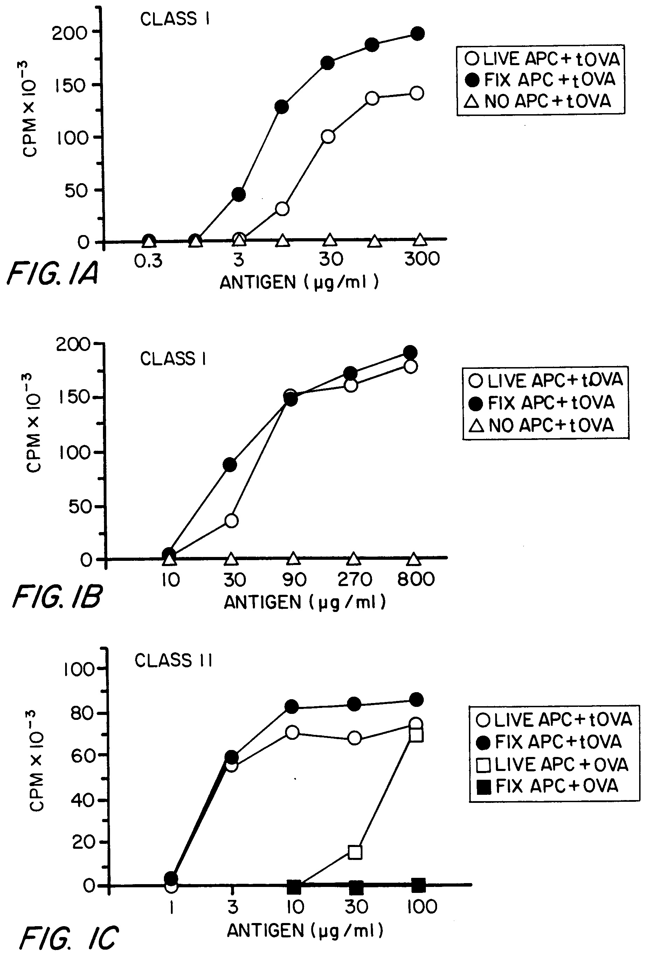

Figure 1 is a graphical illustration which shows that exogenous peptides associate with class I and class II MHC on the surface of antigen presenting cells (APC's). A titration of antigen was added to cultures with T-T hybridomas and either live or fixed APC's or without APC's, as indicated in the legend. In Figure 1A, microcultures were prepared with RF33.70 T-T hybrids (anti-OVA + K^{b}) and EL4 APC's (an H-2^{b}, Class I⁺, Class II⁻ lymphoblastoid cell line); in Figure 1B, with RF33.70 T-T hybrids and LB27.4 APC's (an H-2^{bxd}, Class I⁺, Class II⁺, B lymphoblastoid cell line) and tOVA and in Figure 1C, with 8DO51.15 T-T hybrids (anti-OVA + IA^{d}) with LB27.4 APC's and either native OVA or tOVA. T cell activation was measured using an assay for interleukin-2 production. IL-2 production is expressed as the incorporation of ³ H-thymidine into DNA (CPM x 10⁻³) by HT2 cells cultured in supernatant from the antigen-treated cultures.

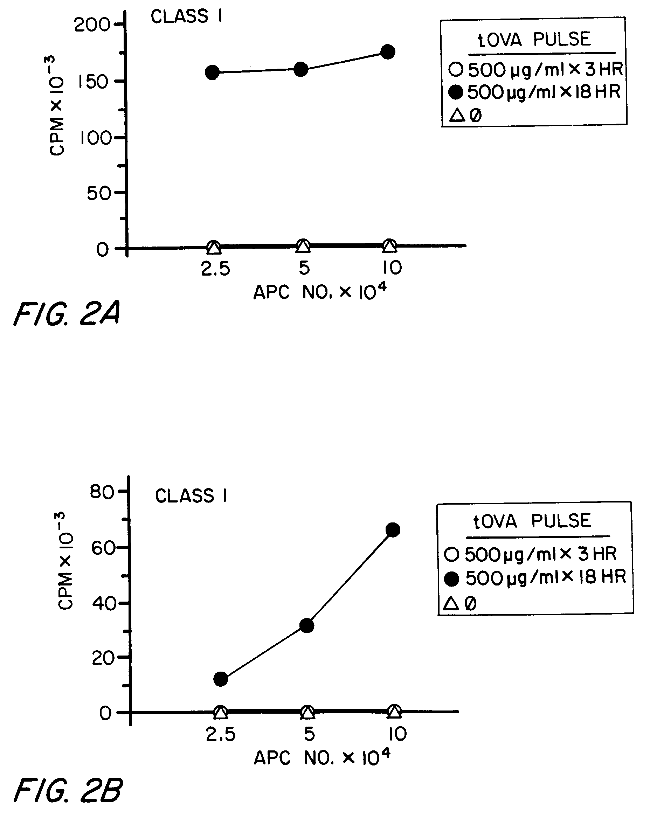

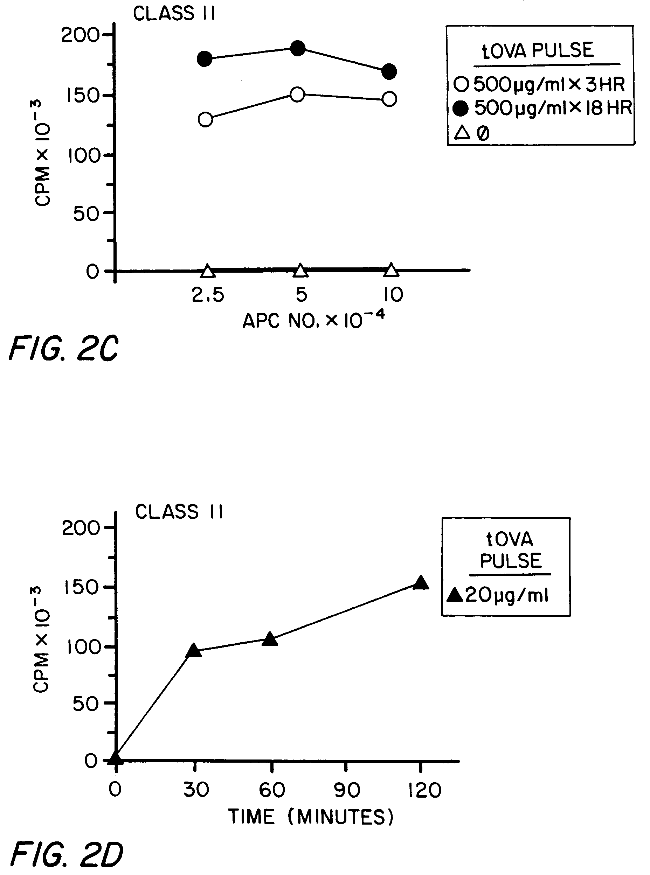

Figure 2 is a graphical illustration of the kinetics of association of exogenous peptides with class I and class II molecules. APC's were preincubated with tOVA for 3 hours or 18 hours and were subsequently fixed and added to cultures with T-T hybridomas. In Figure 2A, microcultures were prepared with RF33.70, OVA + K^{b} specific T-T hybrids with EL4 APC's, in Figure 2B, with RF33.70 T-T hybrids with LB27.4 APC's, in Figure 1C, with 8DO51.15 OVA + IA^{d} specific T -T hybrids with LB27.4 APC's and in Figure 1d, LB27.4 were fixed, incubated with 20µg/ml tOVA for the indicated length of time and added to cultures with DO11.10, OVA + IA^{d} specific T-T hybrids. T cell activation was measured as in Figure 1.

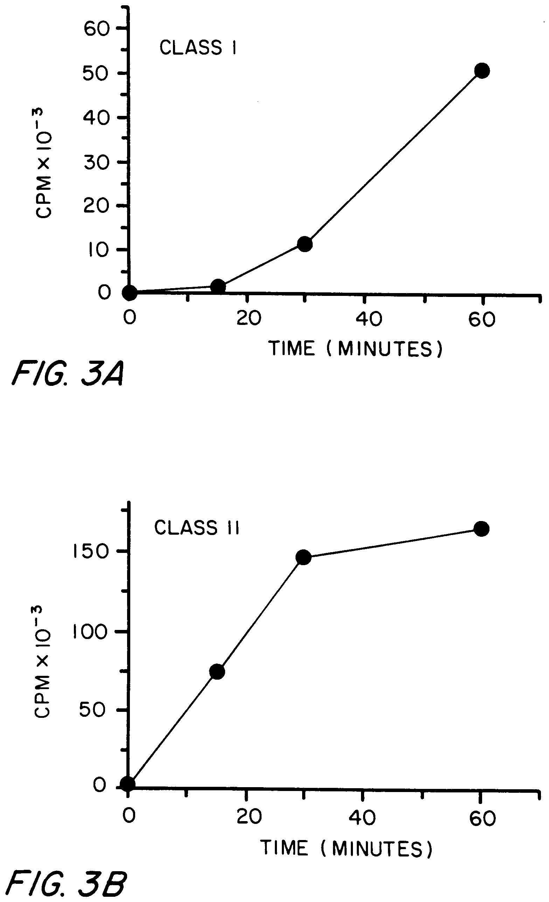

Figure 3 is a graphical illustration which shows that endogenously processed antigens rapidly associate with class I and class II molecules. OVA was introduced into the cytoplasm of LB27.4 APC's by osmotic lysis of pinosomes. The antigen-loaded APC's were incubated at 37°C for the indicated times, fixed and added to cultures with T-T hybrids as follows: In Figure 3A, with RF33.70; in Figure 3B, with DO11.10. T cell activation was measured as in Figure 1 above.

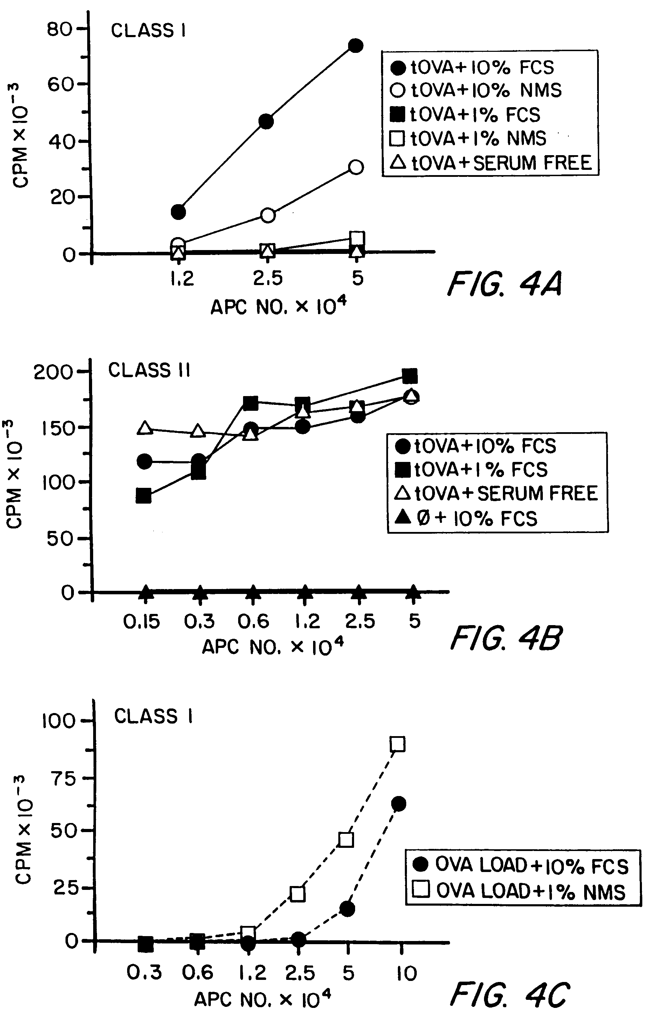

Figure 4 is a graphical illustration which shows that the association of exogenous peptides with class I MHC is uniquely dependent on serum. LB27.4 APC's were incubated with or without tOVA or loaded with OVA by hypotonic lysis of pinosomes. The indicated concentration of normal mouse serum (NMS) or fetal calf serum (FCS) was present during the peptide pulse or in the incubation following the OVA loading. The APC's were subsequently fixed and added to cultures with T-T hybrids as follows: in Figure 4A, with RF33.70 T-T hybrids with tOVA pulsed APC's; in Figure 4B, with DO.11.10 T-T hybrids with tOVA pulsed APC's and in Figure 4C, with RF33.70 cells with OVA loaded APC's. T cell activation was measured as above.

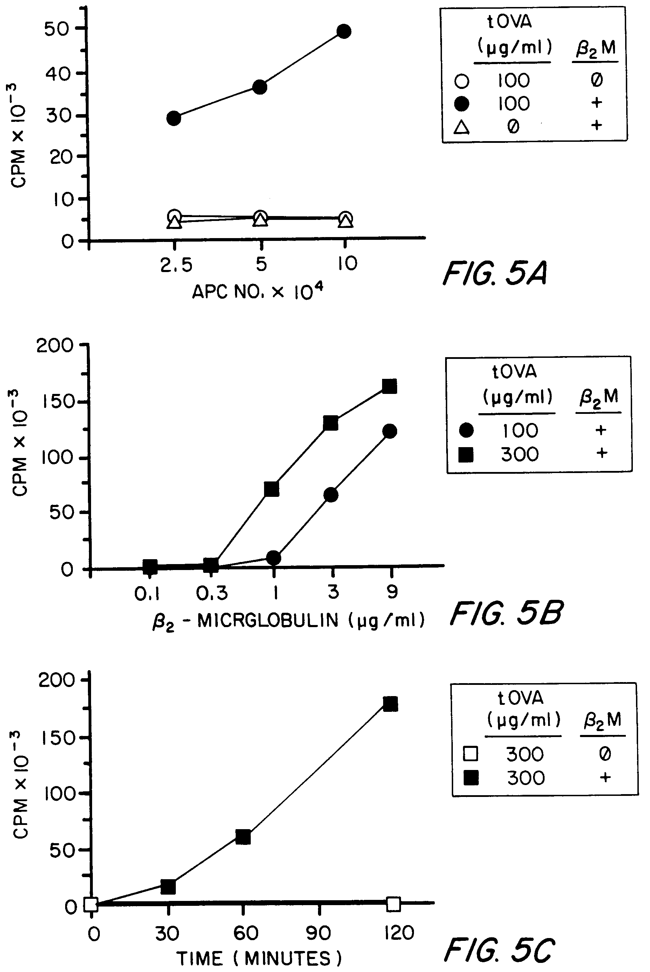

Figure 5 is a graphical illustration which shows that exogenous peptides rapidly associate with class I molecules in the presence of exogenous, purified β₂-microglobulin. APC's were incubated in the presence or absence of the indicated concentrations of β₂-microglobulin. After 2-3 hours incubation at 37°C, the APC's were fixed and added to cultures with the RF33.70, OVA + K^{b} specific T-T hybrid. In Figure 5A, LB27.4 APC's were pulsed with tOVA for 3 hours with or without human β₂-microglobulin; in Figure 5B, EL4 APC's were pulsed for 2 hours with tOVA and human β₂-microglobulin and in Figure 5C, EL4 APC's were pulsed for 0.5-2 hours with tOVA and human β₂-microglobulin.

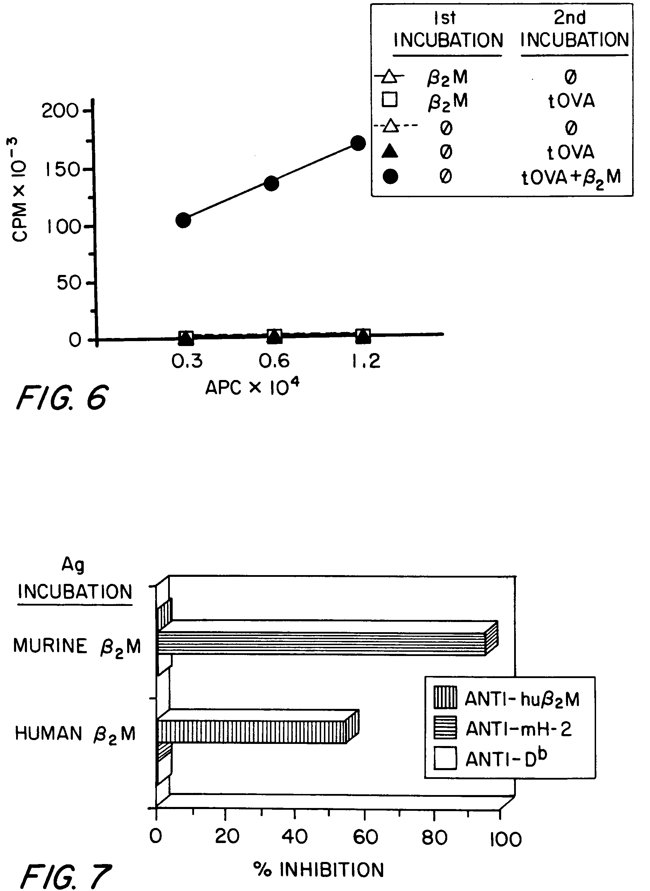

Figure 6 is a graphical illustration demonstrating that exogenous β₂-microglobulin enhances peptide association if present during, but not before peptide pulsing. APC's were incubated with β₂-microglobulin either before or during the peptide pulse with tOVA. Fixed APC's were cultured with RF33.70 T-T hybrids and the supernatant was measured for interleukin 2 content. T cell activation was measured as in the foregoing Figures.

Figure 7 graphically illustrates that exogenous peptide is associated with class I MHC molecules that have undergone β₂-microglobulin exchange. APC's were pulsed with tOVA in media containing exogenous β₂-microglobulin of either murine or human origin. After antigen pulsing, the APC's were incubated with monoclonal antibodies specific for D^{b} (anti-D^{b}), class I MHC molecules with murine β₂-microglobulin (anti-mH-2), or human β₂-microglobulin (anti-huB2M) and were then fixed and added to cultures with RF33.70, OVA + K^{b} specific T-T hybridoma. Percent inhibition was calculated based upon the formula [1-(CPM of experimental group/CPM of control group)] x 100. The experimental groups were anti-mH-2 and anti-huB2M, control group was anti-D^{b}. The response of the control group was 100%.

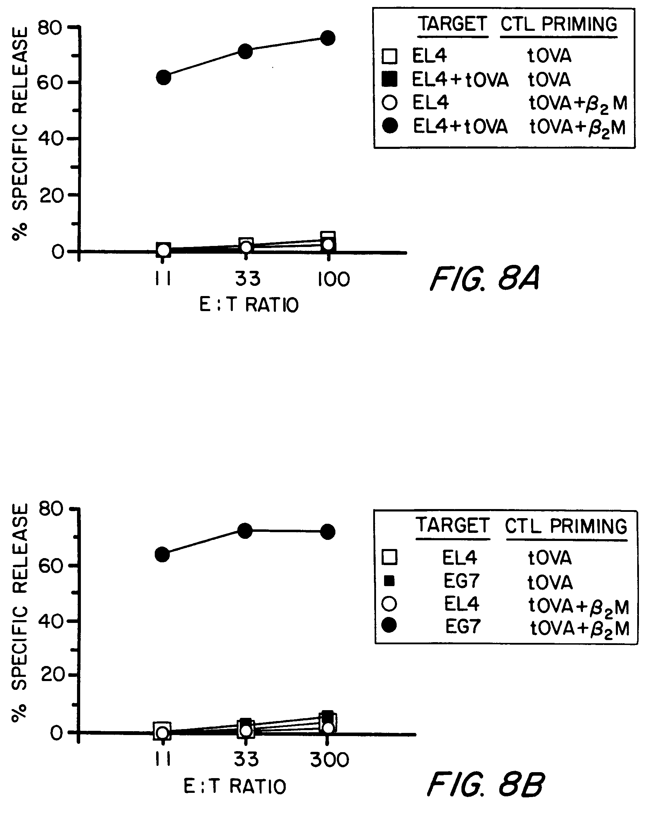

Figure 8 is a graphical illustration demonstrating that effective priming of CTL in vivo can be effected by presenting APC's with peptides and β₂-microglobulin. Individual age and sex matched mice, one mouse per group were injected subcutaneously in each flank (two sites per animal) with 94 µg of tOVA with or without 47µg of purified β₂-microglobulin (Sigma Chemical Co., St. Louis, Mo.) in phosphate buffered saline (47µl per injection site). After 7 days, the mice were sacrificed and the spleens harvested. Splenocytes (30 x 10⁶) were then stimulated with 15 x 10⁶ 20,000 gamma irradiated EG7 cells (a transfected EL4 cell synthesizing OVA) in 10 mls of 10% FCS-containing RPMI 1640 at 37°C. After five days of incubation, the restimulated cells were tested as effectors in a standard ⁵¹ Cr release assay. Targets were, in Figure 8A, EL4 cells (no antigen), or EL4 cells precultured in 300 mg/ml tOVA for 18 hours at 37°C in media with 10% FCS, and in Figure 8B, EG7 cells (a transfected EL4 cell synthesizing OVA). Effector to target ratios were 11, 33 and 100. Data are expressed as % specific release versus effector to target (E:T) ratio.

### Detailed Description of the Invention

The major histocompatibility complex (MHC) is a chromosomal region consisting of a series of genes that code for the cell surface expression of strong transplantation antigens. These transplantation antigens are, in general, glycoproteins that are present on the surface of most nucleated cells. The MHC in mammals is also in the region where the histocompatibility-linked immune response genes are located; hence the chromosomal segment not only controls the synthesis of transplantation antigens and graft rejection, but also influences immune responses to infectious challenge and susceptibility to the development of immunologically mediated diseases. The two MHC systems that have been most extensively characterized are the H-2 system in the mouse and the HLA system in humans.

The MHC's of the mammals studied to date are remarkably similar, suggesting an evolutionary pressure for conservation of these genetic regions. In each instance, the genes code for two general types of antigen; class I and class II. The present invention is generally related to the association of antigen with class I molecules.

The class I genes (HLA-A, B and C in humans, H-2K, D, L in mice) code for multi-determinant antigens which appear on the surface of cells as two peptide chains. Only the heavy chain is coded for by the MHC, and contains hypervariable regions analogous to the immunoglobulins. The heavy chain peptide consists of a large transmembrane glycoprotein of about 44K molecular weight (350 amino acids). This heavy chain is usually non-covalently associated with the light chain, β₂-microglobulin, a 100 amino acid, 12K molecular weight protein. β₂-microglobulin is encoded by genes on a separate chromosome than those coding for the class I heavy chains.

It has been heretofore shown that exchange of β₂- microglobulin chains will occur in the presence of free β₂- microglobulin. Hyafil and Strominger, for example, demonstrated that radio-labeled urinary β₂-microglobulin is incorporated into the human histocompatibility complex HLA upon incubation of the β₂ with papain-solubilized HLA. The radio-labeled urinary β₂-microglobulin was incorporated into HLA, where it was found to have substituted for the pre-existing β₂ that had disassociated from the complex. Proc. Natl. Acad. Sci., U.S.A., Vol. 76, No. 11 pp. 5834-38 (1979). Bernabeu et al demonstrated that β₂-microglobulin from fetal calf serum (FCS) or human serum used in cell culture can exchange β₂-microglobulin associated with HLA antigens and with mouse β₂-microglobulin on the cell surface. Nature, Vol. 308, pp. 642-45 (April 1984). The present invention is based upon the discovery that the binding of exogenous polypeptides to class I MHC molecules on the surface of antigen presenting cells occurs upon disassociation and reassociation of the β₂-microglobulin light chains, which has not heretofore been recognized.

The mechanism of the association of exogenous polypeptides is elucidated in the examples herein, and specifically, in Examples 1-4. These examples demonstrate that class I MHC molecules can associate exogenous polypeptides on the cell surface and can also associate antigen that has been processed intracellularly. The examples also show that, although the class I MHC molecules will associate exogenous polypeptides on the surface of antigen presenting cells, association of peptides with class I MHC is much less efficient than the association of endogenously processed antigen with class I MHC molecules. Elevated levels of free, exogenous β₂-microglobulin have been found, in accordance with the present invention, to be necessary for the efficient association of peptides with class I MHC molecules on the surface of antigen-presenting cells. Although free β₂-microglobulin is inherently present in some serum-containing culture media, it has never been recognized or suggested that a component in the media might facilitate associate peptides with class I molecules. A role for free β₂-microglobulin has heretofore been unknown.

The present invention thus provides a method for markedly enhancing the association of exogenous polypeptides to class I MHC molecules on the surface of antigen presenting cells. The method comprises the steps of obtaining polypeptides of interest and presenting the polypeptides extracellularly to the appropriate antigen presenting cells in the presence of elevated levels of exogenous, free β₂-microglobulin. This method can be utilized to enhance the efficiency of the sensitization of target cells with antigen in vitro, and can also be utilized to prime naive populations of T lymphocytes for antigen-specific cytotoxic T cell response, both in vitro and in vivo. As used herein, the term "sensitize" refers to the association of polypeptides with MHC molecules on antigen presenting cells. The sensitization of class I MHC target or antigen presenting cells is generally determined by assaying for cytotoxic T lymphocyte response using antigen specific CTL's, which recognize antigen in association with the class I restriction pattern. "Priming" refers to the initiation or activation of cytotoxic T lymphocyte response from a naive population of lymphocytes, i.e. a population of T lymphocytes that has not previously been exposed to the antigen. The method of the present invention is useful in the study of class I MHC antigen presentation and cell cell interaction and is also useful for enhancing the immunogenicity of synthetic peptides in vaccines.

The polypeptides that can be used in accordance with the method of the present invention are not particularly limited. As used herein, the words "polypeptide" and "peptide" are used interchangeably and designate a linear sequence of amino acids connected one to the other by peptide bonds between the alpha amino and carboxy groups of adjacent amino acids. The polypeptides can be of a variety of lengths, either in their neutral (uncharged) form or in forms such as their salts and either free of modifications such as glycosylations, side chain oxidation or phosphorylation or containing such modifications. Also included in the definition are polypeptides modified by additional substituents attached to the amino side chains, such as glycosyl units, lipids or inorganic ions such as phosphates as well as chemical modifications of the chains. Thus, the term "polypeptide" or its equivalent is intended to include the appropriate amino acid sequence referenced, subject to the foregoing modifications, which do not destroy its functionality.

In general, the polypeptides used in accordance with the method of the present invention will be useful in research in the area of class I MHC restricted lymphocyte biology, in the development of synthetic class I immunogenic polypeptides that mimic naturally processed antigen in vitro and/or in vivo, and in vaccination with class I synthetic polypeptides designed to elicit a cytotoxic T lymphocyte response. For in vitro applications, the polypeptides can be virtually any exogenous, e.g. extracellularly produced, polypeptide that is of interest. Determination of an appropriate concentration of peptide for in vitro applications is conventional and well within the skill of the art.

For the in vivo applications, the polypeptides will preferably encompass epitopes that can be recognized by class I MHC restricted T lymphocytes. The peptides can be used in the immunization alone, or bound to a carrier or other haptenic molecule. Such polypeptides are sometimes hereinafter referred to as class I polypeptides. As used herein, the term "epitope" refers to that portion of a molecule, usually a protein, that is specifically bound by a T cell receptor or an antibody combining site. The term is also used interchangeably with "antigenic determinant". In most instances, the polypeptide used in accordance with the present invention will be one which has been shown to prime cytotoxic T lymphocyte response in vitro. For example, various synthetic peptides derived from the influenza virus have been shown to induce cytolytic T cell activity in vitro, but not in vivo. These include peptides from the influenza nucleoprotein composed of residues 365-80 (NP365-80), NP50-63, and NP147-58 and peptides from influenza hemagglutinin HA202-21 and HA523-45, defined previously in class I restricted cytotoxicity assays. See, Perkins et al, J. Exp. Med., Vol. 170, 279-289 (July 1989). Enhanced efficiency of association of such polypeptides to class I molecules on antigen presenting cells in vivo has major implications for the use of these synthetic peptides as influenza vaccines. Other examples of synthetic peptides containing known epitopes that can be recognized by CTLs include influenza strain A/Jap/57 hemagglutinin protein, residues 508-530; influenza strain A/PR8/34 nucleoprotein residues 360-385; and HIV Pol (reverse transcriptase) residues 203-219. This list of peptides is exemplary only and is not intended to limit in any way the class I peptides with which the method of the present invention can be employed. Rather, in accordance with the present invention, the association of the class I peptides to class I molecules on the surface of antigen presenting cells can be enhanced, to thereby enhance the immunogenicity of any class I polypeptide immunogen.

The polypeptides used in accordance with the method of the present invention can be prepared in any one of a number of conventional ways. Because they will generally be short sequences, they can be prepared by chemical synthesis using standard techniques. Particularly convenient are the solid phase techniques. Automated peptide synthesizers are commercially available, as are the reagents required for their use. Alternatively, the peptides can be prepared, as in the Examples herein, by enzymatic digestion or cleavage of naturally occurring proteins. The peptides can also be prepared using recombinant techniques known to those of skill in the art.

The source of the β₂-microglobulin employed in accordance with the method of the present invention is not critical. The β₂-microglobulin can thus be obtained, for example, from human, murine, bovine, equine or other mammalian serum or body fluids normally containing β₂-microglobulin. Purified human β₂-microglobulin is available commercially, for example from Sigma Chemical Co., St. Louis, MO., and can be readily obtained. Alternatively, β₂-microglobulin can be isolated and purified from serum or other body fluids using conventional techniques or can be produced by recombinant techniques based upon the introduction of β₂-microglobulin genes into appropriate expression systems. The gene-encoding β₂-microglobulin has previously been cloned. In addition, its sequence is known, thus allowing for the isolation of a DNA clone by persons skilled in the art.

When used as an adjuvant for vaccination with polypeptides, it is preferable, although not necessary, to use β₂-microglobulin originating from the same species as the vaccine recipient in order to reduce the possibility of an immune response to the exogenously produced free β₂-microglobulin.

In accordance with the present invention, an increased number of antigen-class I complexes will occur in the presence of β₂-microglobulin at any point in time. It is expected that more complexes will form at higher concentrations of β₂-microglobulin. More antigen-MHC-class I complexes will result in a stronger and more persistent stimulation, and hence, higher concentrations of β₂-microglobulin are most desirable.

For in vitro applications, the concentration of β₂-microglobulin should be raised to a level higher than that inherently present in serum-containing culture media. For example, cell culture media typically include 10% fetal calf serum, in a 1:10 dilution with another medium, for example RPMI-1640. (Since β₂-microglobulin is present in fetal serum at a level of about 10-fold higher than that present in adult serum, a 10% fetal serum-containing media roughtly approximates conditions in vivo in adult mammalian serum). While the culture media thus contains some free β₂-microglobulin, it has been found in accordance with the present invention, that such concentration is insufficient to cause the efficient association of exogenous peptides with class I MHC molecules. The concentration of free β₂-microglobulin should be increased by at least 2 to 10 fold that normally present in the culture media to achieve the most efficient association in terms of the number of antigen-MHC complexes.

The concentration of free β₂-microglobulin in adult mammalian serum is very low and hence reassociation of free β₂-microglobulin with class I MHC molecules is limited. For in vivo applications, the amount of free β₂-microglobulin must be sufficient to increase the concentration of free β₂-microglobulin in the serum above the level normally present in mammalian serum in order to favor the binding of β₂-microglobulin, and thereby peptides, to class I MHC heavy chains and stimulate an immune response. The concentration of β₂-microglobulin can be elevated systemically, i.e. throughout the body by increased levels in the blood, or locally at the site of injection. The latter is more attractive clinically and economically, and hence is preferred. While the required amount of β₂-microglobulin may vary to some degree from species to species, normal concentration ranges are readily ascertainable and in many instances are available in the published literature. Adult human β₂-microglobulin, for example, is normally present in serum an amount of about 1.5-2 µg/ml. See, "Trace Components of Plasma: Isolation and Clinical Significance," Jamieson, G.A. Ed., Alan R. Liss, Inc. Publisher, New York, N.Y. (1976). The phrase "elevated β₂-microglobulin concentration" thus refers to a concentration that is greater than that normally present in mammalian serum and which is sufficent to stimulate an immune response. Precise amounts of β₂-microglobulin can be tailored to the particular application and peptides used in the immunization.

At high local concentrations of β₂-microglobulin, such as that employed in Example 5 herein, no adverse affects are observed.

In one embodiment of the present invention, the efficiency of the association of exogenous peptides with class I molecules on the surface of antigen presenting cells in vitro can be enhanced by presenting the peptides of interest to the antigen presenting cells in the presence of an elevated concentration of β₂-microglobulin. In the case of sensitization of antigen presenting cells for presentation to antigen-specific cytotoxic T lymphocytes, the antigen presenting cells share class I molecules with the host strain from which the antigen-specific CTL's were developed. The ratio of cytotoxic T lymphocytes (or CTL T-T hybridomas) to antigen presenting cells in the culture is not particularly limited. Generally, there are about 1-100 times the number of CTLs to antigen presenting cells, with a preferred ratio of about 100:1 to about 10:1 for normal CTL populations and 10:1 to ≥ 1:1 for clonal CTL populations. Peptides are presented to the antigen presenting cells in the presence of an elevated level of β₂-microglobulin. Preincubation of APC's with the β₂-microglobulin prior to incubation with the peptides is not sufficient. Both the exogenous peptides and the free β₂-microglobulin must be in the presence of the APC's for enhanced association.

Similarly, in the priming of naive cells with antigen presenting cells in association with peptides, the antigen presenting cells share class I molecules with the strain from which the naive cells were obtained. Naive cells can be, for example, spleen cells from unprimed mice. Other sources of naive T lymphocyte populations include peripheral blood lymphocytes or lymph node cells.

Antigen presenting cells for in vitro applications include macrophages, B lymphocytes, T lymphocytes or the like bearing the appropriate class I MHC molecule. In accordance with this embodiment, the association of peptides with class I molecules on the surface of antigen presenting cells can be enhanced for virtually any exogenous peptide/MHC class I combination.

In another embodiment, β₂-microglobulin may serve as an adjuvant in vaccination of a host organism with exogenous peptides which mimic epitopes of a pathogen against which protection is sought. In accordance with this embodiment, the host organism is immunized with the synthetic peptides and free β₂-microglobulin such that the antigen presenting cells of the host organism are presented with the peptides and β₂-microglobulin. When administered in effective amounts in the presence of elevated levels of free β₂-microglobulin, the peptides are capable of initiating effector and memory class I restricted cytotoxic T lymphocytes which immunoreact with the polypeptides or with pathogen related immunogen that corresponds in amino acid sequence to the exogenous polypeptides used in the vaccine on the surface of antigen presenting cells. In accordance with the method of the present invention, it is possible to achieve more peptide/class I MHC complexes and therefore stronger stimulation of CTL response than with other known methods.

The effective amount of exogenous polypeptide in a unit dose depends upon a number of factors. Included among these factors are the nature of the carrier, if one is used, the amount of β₂-microglobulin, the number of inoculations to be used and, to some extent, the body weight of the mammal to be immunized. Individual local inoculations will typically contain about 50 micrograms to about 5 milligrams of polypeptide exclusive of any carrier to which the peptides may be linked. For systemic administration, inoculations may typically contain about 0.5 to 3 mg/kg of body weight. To achieve a useful level of protection in a population with synthetic peptides, a variety of peptide epitopes, derived from various subunits of the pathogen preferably will preferably be employed, since MHC molecules are known to be polymorphic. Alternatively, where a specific peptide is known to be reactive with a particular MHC class I molecule, individuals with that MHC-specificity can be specifically immunized with the peptide. In addition to the class I restricted peptides, the vaccine can additionally include class II MHC-restricted peptides, the latter to supply help for the CTL response.

The preparation of vaccines which contain peptide sequences as active ingredients is well understood in the art. Typically, such vaccines are prepared as injectables, either as liquid solutions or suspensions. The active ingredients are often mixed with excipients which are pharmacologically acceptable and compatible with the active ingredient. Suitable excipients are, for example, water, saline, dextrose, glycerol, ethanol, or the like and combinations thereof, with saline being particularly preferred. In addition, and in accordance with the present invention, the vaccine contains free β₂-microglobulin as an adjuvant which enhances the effectiveness of the vaccine.

The vaccines are conventionally administered by injection, for example either subcutaneously or intramuscularly. In accordance with the present invention, the exogenous peptides which are the active ingredients of the vaccine are presented to antigen presenting cells of the immune system in the presence of elevated levels of free β₂-microglobulin. Thus, in one embodiment, the exogenous peptides and free β₂ can be prepared as an injectable and simultaneously injected into the mammal to be immunized. The peptides and β₂ can either be free in the liquid solutions or suspensions or can be encapsulated in a depot or other controlled release vehicle. Alternatively, the exogenous peptides and free β₂ can be injected sequentially. Booster injections can be given, if needed. The critical feature is that the peptides are presented to the APC's in the presence of free β₂-microglobulin.

The present invention will be more clearly understood from the following specific examples.

### Example 1

### Association of Exogenous Peptides With Class I MHC Molecules

Although it has been generally assumed that exogenous peptides associate directly with class I MHC molecules at the cell surface, this assumption has not been formally tested in most systems, because conventional cytolytic assays require intact antigen presenting cells. There is recent evidence, however, that exogenously added peptides may associate intracellularly with newly synthesized class I molecules. This example demonstrates that peptides can associate with class I MHC molecules at the APC cell surface.

Microcultures were prepared with 5-10 x 10⁴ T-T hybridomas with a titration of tOVA and with or without 5 x 10⁴ live or fixed antigen presenting cells in RPMI 1640-10% fetal calf serum (FCS) media. The T-T hybridomas were RF33.70, an anti-OVA + K^{b} hybrid which secretes interleukin-2 upon appropriate antigenic stimulation, and 8DO51.15, an anti-OVA + IA^{d} hybrid. Antigen presenting cells were EL4 cells, (an H-2^{b}, Class I+, Class II-, T lymphoblastoid cell line) or LB27.4 cells, an H-2^{bxd}, Class I⁺, Class II+, lymphoblastoid cell line. The antigen presenting cells were chemically fixed with glutaraldehyde as previously described. Shimonkevitz et al., J. Exp. Med., Vol. 158, pp. 303-16 (1983).

A titration of exogenous peptides, tOVA, as set forth in the legend to Figures 1 A-C herein, was added to the cultures and the cultures were incubated for 18 hours at 37°C. After 18 hours, a 100µl aliquot of supernatant was removed and assayed for interleukin-2 (IL-2) content with HT2 cells as previously described. Rock et al., J. Exp. Med., Vol. 157, pp. 1618-34 (1983). As illustrated in Figure 1, exogenous peptides associate with class I and class II molecules on the surface of antigen presenting cells.

As also illustrated in Figure 1, fixed APC's present tOVA to RF33.70 (Figures 1A and B). The chemical fixation in this example is adequate because the treated APC's are unable to process native antigen for presentation with class II (Fig. 1C) or class I (Fig. 3) MHC molecules and were otherwise metabolically inactive. These results demonstrate that peptides can associate with K^{b} molecules on the antigen presenting cell surface.

### Example 2

### Kinetics of Peptide-MHC Association

This example examines the kinetics of formation of peptide-MHC complexes with class I versus class II molecules.

Live or fixed antigen presenting cells were preincubated with tOVA for 3 or 18 hours at the concentrations indicated in Figure 2 and were subsequently fixed and added to cultures with T-T hybridomas as follows.
A. RF33.70, OVA + K^{b} T-T hybridoma with EL4 APC's
B. RF33.70 T-T hybrids with LB27.4 APC's
C. 8DO51.15, OVA + IA^{d} specific T-T hybrids with LB27.4 APC's and
D. LB27.4 APC's were fixed, incubated with 20µg/ml t OVA and added to cultures with DO.11.10, OVA + IA^{d} specific T cells.

Live or fixed APC's were incubated with the indicated concentration of tOVA in RPMI 1640 culture media with 10% fetal calf serum. After 3 or 18 hours, the APC's were washed and the live APC's were fixed. Microcultures were prepared with 2-5 x 10⁵ (A-C) or 5 x 10⁴ (D) fixed APC's and 5 x 10⁵ T-T hybrids. Antigen presenting cells were fixed with paraformaldehyde as previously described. Falo, et al., Proc. Natl. Acad. Sci., U.S.A., Vol. 82, pp. 6647-51 (1985). After 20 hours of incubation at 37°C, a 100 µl aliquot of supernatant was removed and assayed for IL-2 content.

A marked difference in the behaviour of class I versus class II molecules was observed. The association of tOVA with Ia^{d} molecules (class II) on LB27.4 APC's occurs rapidly (Fig. 2C) even at low peptide concentrations and when the APC's are fixed (Fig. 2D) which indicates that there are Ia molecules on the cell surface that are highly receptive to exogenous peptides. In contrast, the association of tOVA with class I MHC molecules on the same APC's requires extended incubation in relatively high concentrations of peptide (Fig. 2B). The slow association between exogenous OVA peptide and K^{b} molecules appears to be a general phenomenon, as it is observed with both B (Fig. 2B) and T (Fig. 2A) APC's. The EL4 APC's are more active than LB27.4, which may reflect the higher level of expression of K^{b} on the former. This example thus shows that exogenous peptides associate much more readily with class II molecules than class I molecules on the surface of APC's.

### Example 3

### Endogenous Peptide Class I MHC Association

This example demonstrates the kinetics of formation of peptide-MHC complexes arising from endogenously produced antigen. OVA was introduced into the cytoplasm of LB27.4 APC's by osmotic lysis of pinosomes, as previously described. See, Moore, et al., Cell, Vol. 54, pp. 777-85 (1988); Okada, et al., Cell, Vol. 29, pp. 33-41 (1982). To summarize, LB27.4 APC's were incubated with 30mg/ml OVA in hypertonic buffer for 10 minutes at 37°C, followed by a 3 minute incubation in hypertonic buffer and were then washed at 4°C. The OVA loaded APC's were incubated at 37°C for 0-60 minutes and were then fixed with paraformaldehyde. Microcultures were prepared with 10 x 10⁴ or 1.25 x 10⁴ APC's and 5-10 x 10⁴ T-T hybridomas. T-T hybrids were RF33.70 and DO.11.10, respectively. After 20 hours incubation, 100 µl aliquots of supernatant were collected and assayed for IL-2 content as previously described.

The example shows the appearance of OVA-K^{b} complexes on the APC surface is detectable after 15-30 minutes and is near maximal by 1 hour following antigen loading (Fig 3A). The rapid appearance of antigen-class I MHC complexes is observed even under limiting conditions of antigen. It should be noted that the ability of the OVA-loaded APC's to present antigen required incubation at 37°C and was inhibited by fixation, which presumably reflects a requirement for intracellular processing or transport. These results indicate that peptide-class I MHC complexes can form rapidly, which resembles the behavior of class II molecules (Fig. 3B) more closely than that of the mature class I MHC molecules on the APC surface.

### Example 4

### B₂- microglobulin and Peptide Association

a. The association of exogenous peptide with class I molecules is uniquely dependent on serum. LB27.4 APC's were either incubated with or without tOVA or loaded with OVA by the hypotonic lysis of pinosomes. The APC's in this example were adapted to grow in serum free media (SFM)(OPTIMEM media, GIBCO, Grand Island, N.Y.) supplemented with 1% SP-nutridoma (Boehringer Mannheim, Indianapolis, IN) supplemented with 1% normal mouse serum (NMS). APC's were incubated for 18 hours at 37°C, with (a) 400µg/ml or (b) 100 µg/ml tOVA in SFM supplemented with 1 or 10% normal mouse serum (NMS) or fetal calf serum or were loaded with OVA (20mg/ml) via osmotic lysis of pinosomes and were incubated for 1 hour at 37°C in SFM supplemented with 1% NMS or 10% FCS. The APC's were subsequently fixed with paraformaldehyde. Microcultures were prepared with 5-10 x 10⁴ T-T hybridomas and the number a APC's, as indicated in Figure 4. The microcultures were handled as previously described and assayed for IL-2 content.
   The results show that the formation of peptide-class I MHC complexes, that occurs upon extended incubation of APC's with exogenous peptide, is markedly influenced by the exogenous serum concentration. As shown in Fig. 4A, tOVA associates with class I in the presence of 10%, but not in 1% serum. The critical variable is the concentration of serum, rather than the species of origin of the serum, although the heterologous fetal serum may be more active. Moreover, when the same APC's are loaded with native OVA, the generation of OVA-class I MHC complexes from endogenously processed antigen and intracellular class I molecules is not affected by the exogenous serum (Fig. 4C).
   In contrast to class I, the association of peptides with class II molecules on the surface of APC's is not dependent on the exogenous serum (Fig 4B). These results indicated that a component in serum may be necessary or facilitate peptide association uniquely with class I MHC molecules on the APC surface.
b. The effect of exogenous β₂-microglobulin. This example demonstrates that the efficiency of association of exogenous peptide with K^{b} class I MHC can be significantly enhanced by increasing the amount of free β₂-microglobulin present in the incubation media. EL4 or LB27.4 APC's were pulsed with tOVA in media containing low amounts of serum and in the presence or absence of purified human β₂-microglobulin as follows.
   APC's were incubated with or without 100 or 300 µg/ml tOVA in complete SFM, 1% NMS, as described above, and with the addition of purified β₂-microglobulin (Sigma Chemical Co., St. Louis, MO.) at (a) 2.5 µg/ml, (b) 0.1-9 µg/ml and (c) 9 µg/ml. After 2-3 hours of incubation at 37°C, the APC's were fixed with paraformaldehyde and added to cultures with the RF33.70, OVA-K^{b}. specific T-T hybridomas. Microcultures were prepared with 5-10 x 10⁴ APC's and handled as previously described.
   The results of this study demonstrate that peptide rapidly associates with class I MHC molecules in the presence of exogenous, purified β₂-microglobulin. In the presence of exogenous β₂-microglobulin, there is extremely effective pulsing of class I MHC molecules on both APC's (Fig. 5). Under these conditions, the association of peptide is rapid and can be detected after 30-60 minutes (Fig. 5C). The optimal concentration of β₂-microglobulin under these conditions is greater than 9 µl/ml. At low concentrations, such as are present in typical culture media containing 10% serum, the formation of OVA-class I molecules is not detectable in this time period (Fig. 5B). The species of β₂-microglobulin is not critical, as evidenced, for example, by Figures 4A and 5. Thus, in accordance with the present invention, the association of peptide with class I MHC molecules on the surface of antigen presenting cells can be enhanced by increasing the amount of free β₂-microglobulin to a concentration greater than that normally found in culture media. Since fetal serum contains up to 10 times more β₂-microglobulin than adult serum, the amount of β₂ in media supplemented with 10% serum approximates the conditions in vivo.
c. Preincubation of APC's with β₂-microglobulin. This example demonstrates that the association of exogenous peptides with class I molecules requires that the exogenous β₂-microglobulin be present at the time of peptide pulsing. APC's were incubated with β₂-microglobulin either before or during pulsing with the exogenous peptide, tOVA. EL4 APC's were incubated with or without 2.5 µg/ml of purified human β₂-microglobulin for 18 hours at 37°C and then washed. The APC's were subsequently incubated with or without 200 µg/ml tOVA in SFM, 1% NMS, in the presence or absence of 2.5 µg/ml human β₂. After 3 hours incubation at 37°C, the APC's were fixed with paraformaldehyde. Micro-cultures were prepared as described in Example 4a above.
   As illustrated in Figure 6, APC's that have been preincubated with purified β₂-microglobulin, washed and then incubated with peptide for three hours do not present tOVA in association with class I molecules. The association of tOVA with K^{b} requires that the exogenous β₂-microglobulin be present at the time of peptide pulsing.
   The results of this study demonstrate that exogenous β₂-microglobulin enhances peptide association if present during, but not before, peptide pulsing with exogenous peptide.
d. Exogenous peptide is associated with class I MHC molecules that have undergone β2-microglobulin exchange. This example indicates that binding of exogenous peptide occurs during the exchange of MHC β₂-microglobulin with exogenous β₂-microglobulin. APC's were pulsed with tOVA in media containing exogenous β₂-microglobulin of human or murine origin. After antigen pulsing, the APC's were incubated with monoclonal antibodies (MAb's) specific for D^{b} (Anti-D^{b}), class I MHC molecules with murine β2- microglobulin (anti-mH-2) or human β₂-microglobulin (anti-huB2-M), and were then fixed and added to cultures with the RF33.70, OVA + K^{b} specific T-T hybridoma.

Specifically, LB27.4 APC's were incubated with 400µg/ml tOVA in SFM supplemented with either 10% NMS (as a source of murine β₂-microglobulin) or 1% NMS and 2.5 µg/ml human β₂-microglobulin for 18 hours at 37°C. The antigen pulsed APC's were washed, incubated in a saturating amount of 28.14.8S (anti-D^{b}), M1/42 (anti-murine class I heterodimers) or BBM.1 (anti-human β₂-microglobulin), for 30 minutes at 4°C, and then were fixed. Microcultures were prepared with 1 x 10⁵ NMS cultured APC's or 3 x 10⁴ human β₂ cultured APC's and 10⁵ T-T hybrids and were handled as described in Example 1. No additional MAb was added to these cultures. The APC's pulse more strongly in the presence of human β₂-microglobulin and therefore the number of APC's added to the cultures was adjusted to give approximately equivalent responses. Under these conditions, the response of the RF33.70 hybrid to APC's pulsed with NMS and human β₂-microglobulin was 35 x 10³ counts per minute (CPM) and 41 x 10³ (CPM), respectively, in the control (28.14.8S) groups. The response of the APC's not exposed to antigen was 0.4 x 10³ CPM.

The results show that T cell responses to APC's pulsed with tOVA and mouse β₂-microglobulin are inhibited by the M1/42 MAb, that is specific for murine-murine, class I heterodimers, and are unaffected by the BBM.1 MAb, that is specific for human β₂-microglobulin (Fig. 7). In contrast, a reciprocal pattern of inhibition was observed when the same APC's were pulsed with tOVA in the presence of exogenous human β₂-microglobulin (Fig. 7). The inhibition that was observed with the anti-human β₂-microglobulin MAb was only partial. However, this does not reflect the presence of peptide bound to murine-murine class I heterodimers, since the M1/42 MAb does not cause inhibition. The partial effect of the BBM.1 MAb could reflect the location of the bound MAb (distant from the peptide/T cell receptor interaction sites), elution of the bound MAb and/or exchange with bovine β₂-microglobulin. Taken together, these results indicate that the association of exogenous peptides with class I molecules on the cell surface requires the disruption and reformation of the class I MHC heterodimer.

### Example 5

### In Vivo Immunization

This experiment establishes that the method of the present invention can be utilized to induce cytotoxic T lymphocyte response in vivo.

Individual age and sex matched C57BL6 mice, one per group, were injected subcutaneously in each flank (two sites per animal) with 94 µg of tOVA with or without 47µg of purified β₂-microglobulin (Sigma Chemical Co., St. Louis, Mo.) in phosphate buffered saline (47µl per injection site). The total amount of tOVA injected into each animal was thus 188 µg; total β₂-microglobulin was 94 µg. After 7 days, the mice were sacrificed and the spleens harvested. Splenocytes (30 x 10⁶) were then stimulated with 15 x 10⁶ 20,000 gamma irradiated EG7 cells (a transfected EL4 cell synthesizing OVA) in 10 mls of 10% FCS-containing RPMI 1640 at 37°C.

After five days of incubation, the restimulated cells were tested as effectors in a standard ⁵¹ Cr release assay. Targets were EL4 cells (no antigen), EL4 cells precultured in 300 mg/ml tOVA for 18 hours at 37°C in media with 10% FCS, or EG7 cells (a transfected EL4 cell synthesizing OVA).

As illustrated in Figures 8A and B, injection of tOVA and β₂-microglobulin into unprimed mice induced a good cytotoxic T lymphocyte response; restimulated spleen cells lysed EL4 APC's in the presence of tOVA, but not in the presence of APC's alone. Consistent with previous reports in the literature (See, Carbone and Bevan, J. Exp. Med., Vol. 169, pp. 603-11 (March 1989)), no priming was obtained with tOVA in the absence of β₂-microglobulin. This control establishes the criticality of an elevated level of β₂-microglobulin for in vivo priming with peptides. Figure 8B illustrates that priming with peptides and β₂-microglobulin stimulated CTL that can recognize the naturally produced/ processed form of OVA (EG7 cells), which is important for vaccine applications.

The results of this study demonstrate that normal APC's exposed in vivo to exogenous peptide in the presence of an elevated level of β₂-microglobulin will acquire the peptides in a manner which allows for the priming of antigen specific cytotoxic T lymphocytes.

From the above description, it is apparent that the objects of the present invention have been achieved. While only certain illustrative embodiments have been set forth, alternative embodiments will be apparent from the above description to those skilled in the art.

## Claims

1. Use of β₂-microglobulin for the manufacture of a therapeutic agent to be used in a method for enhancing the association of exogenous peptide with class I MHC molecules on the surface of antigen presenting cells comprising the steps of:
(a) obtaining the exogenous peptides and
(b) presenting the exogenous peptides to the antigen presenting cells in the presence of an elevated amount of free β₂-microglobulin sufficient to stimulate an immune response, corresponding to a level higher than that normally present as free β₂-microglobulin in mammalian serum.

2. Use of β₂-microglobulin according to claim 1, wherein the β₂-microglobulin is selected from the group consisting of purified human β₂-microglobulin, purified murine β₂-microglobulin, purified bovine β₂-microglobulin and purified equine β₂-microglobulin and mixtures thereof.

3. Use of β₂-microglobulin according to claim 2, wherein the β₂-microglobulin is purified human β₂-microglobulin.

4. Use of β₂-microglobulin according to anyone of claims 1 to 3, wherein the exogenous peptides comprise complete trypsin-digested ovalbumin peptides.

5. Use of β₂-microglobulin according to anyone of claims 1 to 4, wherein the exogenous peptides comprise cyanogen-bromide cleaved ovalbumin peptides.

6. Use of β₂-microglobulin according to anyone of claims 1 to 5, wherein the exogenous peptide is an immunogenic epitope for a pathogen.

7. Use of β₂-microglobulin for the manufacture of a therapeutic agent to be used in a method of priming class I MHC-restricted cytotoxic T lymphocyte response with exogenous peptides comprising the steps of
(a) obtaining the exogenous peptides and
(b) presenting exogenous peptides to antigen presenting cells bearing class I MHC molecules in the presence of an amount of free β₂-microglobulin and culturing the thus-prepared cells in the presence of a native population of T lymphocytes wherein the β₂-microglobulin is present in an amount sufficient to stimulate an immune response and at a level higher than that normally present as free β₂-microglobulin in mammalian serum.

8. Use of β₂-microglobulin according to claim 7, wherein the β₂-microglobulin is selected from the group consisting of purified human β₂-microglobulin, purified murine β₂-microglobulin, purified bovine β₂-microglobulin and purified equine β₁-microglobulin and mixtures thereof.

9. Use of β₂-microglobulin according to claim 8, wherein the exogenous peptides are presented to antigen presenting cells bearing class I MHC molecules in the presence of an elevated amount of free β₂-microglobulin in vivo.

10. Use of β₂-microglobulin for the manufacture of a therapeutic agent to be used in a method of using purified β₂-microglobulin as an adjuvant for a synthetic peptide vaccine comprising the steps of: obtaining purified β₂-microglobulin and presenting synthetic polypeptides comprising pathogen-derived epitopes to mammalian host immune cells in the presence of elevated levels of free β₂-microglobulin wherein the β₂-microglobulin is present in an amount sufficient to stimulate an immune response and at a level higher than that normally present as free β₂-microglobulin in mammalian serum.

11. Use of β₂-microglobulin according to claim 10, wherein the synthetic peptides and free β₂-microglobulin are simultaneously injected into the mammalian host.

12. Use of β₂-microglobulin according to claim 10, wherein the synthetic peptides and free β₂-microglobulin are sequentially injected into the mammalian host.

13. Use of β₂-microglobulin for the manufacture of a therapeutic agent to be used in a method of inducing class I MHC-restricted cytotoxic T lymphocyte response to a pathogen-related immunogen in a mammalian host comprising the steps of
(a) obtaining a pathogen-related immunogen and
(b) exposing a naive population of T lymphocytes to the pathogen-related immunogen in the presence of free β₂-microglobulin, said β₂-microglobulin being present in a concentration greater than the concentration of β₂-microglobulin normally present in mammalian serum and sufficient to cause an immune response.

14. Use of β₂-microglobulin according to claim 13, wherein the β₂-microglobulin is selected from the group consisting of purified human β₂-microglobulin, purified murine β₂-microglobulin, purified bovine β₂-microglobulin, purified equine β₂-microglobulin and mixtures thereof.

15. Use of β₂-microglobulin according to claim 14, wherein the β₂-microglobulin is purified human β₂-microglobulin.

16. Use of β₂-microglobulin according to anyone of claims 13 to 15, wherein the free β₂-microglobulin is introduced into the mammalian host in an amount that is from about 2 to about 10 fold higher than that normally present in the serum of said mammalian host.

## Patentansprüche

1. Verwendung von β₂-Mikroglobulin zur Herstellung eines therapeutischen Mittels für die Anwendung in einem Verfahren zur Verbesserung der Assoziation exogener Peptide mit MHC-Molekülen der Klasse I auf der Oberfläche von antigentragenden Zellen mit den Schritten:
(a) Gewinnen der exogenen Peptide und
(b) Darbieten der exogenen Peptide den antigentragenden Zellen in Gegenwart einer erhöhten Menge an freiem β₂-Mikroglobulin, das ausreicht, eine Immunantwort zu stimulieren, entsprechend einem Niveau, das höher als dasjenige ist, das normalerweise als freies β₂-Mikroglobulin in Säugetierserum vorhanden ist.

2. Verwendung von β₂-Mikroglobulin nach Anspruch 1, wobei das β₂-Mikroglobulin gereinigtes menschliches β₂-Mikroglobulin, gereinigtes Mäuse- oder Ratten-β₂-Mikroglobulin, gereinigtes Rinder-β₂-Mikroglobulin oder gereinigtes Pferde-β₂-Mikroglobulin oder eine Mischung hiervon ist.

3. Verwendung von β₂-Mikroglobulin nach Anspruch 2, wobei das β₂-Mikroglobulin gereinigtes menschliches β₂-Mikroglobulin ist.

4. Verwendung von β₂-Mikroglobulin nach einem der Ansprüche 1 bis 3, wobei die exogenen Peptide vollständige Trypsin-verdaute Ovalbumin-Peptide umfassen.

5. Verwendung von β₂-Mikroglobulin nach einem der Ansprüche 1 bis 4, wobei die exogenen Peptide mit Zyanbromid gespaltene Ovalbumin-Peptide umfassen.

6. Verwendung von β₂-Mikroglobulin nach einem der Ansprüche 1 bis 5, wobei das exogene Peptid ein immunogenes Epitop für einen Krankheitsverursacher ist.

7. Verwendung von β₂-Mikroglobulin zur Herstellung eines therapeutischen Mittels für die Anwendung in einem Verfahren zum priming eines Klasse-I-MHC-beschränkten zytotoxischen T-Lymphozyt-Ansprechverhaltens mit exogenen Peptiden mit den Schritten:
(a) Gewinnen der exogenen Peptide und
(b) Darbieten exogener Peptide den antigentragenden Zellen, welche MHC-Moleküle der Klasse I tragen, in Gegenwart einer Menge an freiem β₂-Mikroglobulin und Anlegen einer Kultur der so vorbereiteten Zellen in Gegenwart einer nativen Population von T-Lymphozyten, wobei das β₂-Mikroglobulin in einer Menge vorhanden ist, die ausreicht, eine Immunantwort zu stimulieren und bei einem Niveau, das höher als dasjenige ist, das normalerweise als freies β₂-Mikroglobulin in Säugetierserum vorhanden ist.

8. Verwendung von β₂-Mikroglobulin nach Anspruch 7, wobei das β₂-Mikroglobulin gereinigtes menschliches β₂-Mikroglobulin, gereinigtes Mäuse- oder Ratten-β₂-Mikroglobulin, gereinigtes Rinder-β₂-Mikroglobulin oder gereinigtes Pferde-β₂-Mikroglobulin oder eine Mischung hiervon ist.

9. Verwendung von β₂-Mikroglobulin nach Anspruch 8, wobei die exogenen Peptide den antigentragenden Zellen, welche MHC-Moleküle der Klasse I tragen, in Gegenwart einer erhöhten Menge an freiem β₂-Mikroglobulin in vivo dargeboten werden.

10. Verwendung von β₂-Mikroglobulin zur Herstellung eines therapeutischen Mittels für die Anwendung in einem Verfahren zur Benutzung gereinigten β₂-Mikroglobulins als ein Adjuvans für einen synthetischen Peptid-Impfstoff mit den Schritten: Gewinnen gereinigten β₂-Mikroglobulins und Darbieten synthetischer Polypeptide, die von Krankheitsverursachern abgeleitete Epitope umfassen, gegenüber Säugetier-Wirtsimmunzellen in Gegenwart erhöhter Niveaus an freiem β₂-Mikroglobulin, wobei das β₂-Mikroglobulin in einer Menge anwesend ist, die ausreicht, eine Immunantwort zu stimulieren und bei einem Niveau, das höher als dasjenige ist, welches normalerweise als freies β₂-Mikroglobulin in Säugetierserum vorhanden ist.

11. Verwendung von β₂-Mikroglobulin nach Anspruch 10, wobei die synthetischen Peptide und freies β₂-Mikroglobulin gleichzeitig in den Säugetierwirt injiziert werden.

12. Verwendung von β₂-Mikroglobulin nach Anspruch 10, wobei die synthetischen Peptide und freies β₂-Mikroglobulin nacheinander in den Säugetierwirt injiziert werden.

13. Verwendung von β₂-Mikroglobulin zur Herstellung eines therapeutischen Mittels für die Anwendung in einem Verfahren zur Induzierung eines Klasse-I-MHC-beschränkten zytotoxischen T-Lymphozyten-Ansprechverhaltens auf ein auf einen Krankheitsverursacher bezogenes Immunogen in einem Säugetierwirt mit den Schritten:
(a) Gewinnung eines auf einen Krankheitsverursacher bezogenen Immunogens und
(b) Exponieren einer nativen Population von T-Lymphozyten gegenüber dem auf einen Krankheitsverursacher bezogenen Immunogen in Gegenwart von freiem β₂-Mikroglobulin, wobei das β₂-Mikroglobulin in einer Konzentration zugegen ist, die größer ist als die Konzentration an β₂-Mikroglobulin, wie sie normalerweise in Säugetierserum vorhanden ist und ausreicht, eine Immunantwort hervorzurufen.

14. Verwendung von β₂-Mikroglobulin nach Anspruch 13, wobei das β₂-Mikroglobulin gereinigtes menschliches β₂-Mikroglobulin, gereinigtes Mäuse- oder Ratten-β₂-Mikroglobulin, gereinigtes Rinder-β₂-Mikroglobulin oder gereinigtes Pferde-β₂-Mikroglobulin oder eine Mischung hiervon ist.

15. Verwendung von β₂-Mikroglobulin nach Anspruch 14, wobei das β₂-Mikroglobulin gereinigtes menschliches β₂-Mikroglobulin ist.

16. Verwendung von β₂-Mikroglobulin nach einem der Ansprüche 13 bis 15, wobei das freie β₂-Mikroglobulin in den Säugetierwirt in einer Menge eingeführt wird, die um etwa das Zwei- bis etwa das Zehnfache höher als diejenige ist, die normalerweise im Serum des Säugetierwirts vorhanden ist.

## Revendications

1. Utilisation d'une β₂-microglobuline pour la préparation d'un agent thérapeutique destiné à être utilisé dans un procédé pour renforcer l'association d'un peptide exogène avec des molécules CMH de la classe I sur la surface de cellules présentant des antigènes, comprenant les étapes suivantes :
(a) obtention des peptides exogènes et
(b) présentation des peptides exogènes aux cellules présentant des antigènes en présence d'une quantité élevée de β₂-microglobuline libre, suffisante pour stimuler une réponse immune correspondant à un niveau supérieur à celui normalement présent sous forme de β₂-microglobuline libre dans le sérum de mammifère.

2. Utilisation d'une β₂-microglobuline selon la revendication 1, dans laquelle la β₂-microglobuline est choisie dans le groupe composé par la β₂-microglobuline humaine purifiée, la β₂-microglobuline murine purifiée, la β₂-microglobuline bovine purifiée et la β₂-microglobuline équine purifiée, et leurs mélanges.

3. Utilisation d'une β₂-microglobuline selon la revendication 2, dans laquelle la β₂-microglobuline est une β₂-microglobuline humaine purifiée.

4. Utilisation d'une β₂-microglobuline selon l'une quelconque des revendications 1 à 3, dans laquelle les peptides exogènes comprennent des peptides d'ovalbumine complètement digérée par une trypsine.

5. Utilisation d'une β₂-microglobuline selon l'une quelconque des revendications 1 à 4, dans laquelle les peptides exogènes comprennent des peptides d'ovalbumine coupée par du bromure de cyanogène.

6. Utilisation d'une β₂-microglobuline selon l'une quelconque des revendications 1 à 5, dans laquelle le peptide exogène est un épitope immunogénique pour un agent pathogène.

7. Utilisation d'une β₂-microglobuline pour la préparation d'un agent thérapeutique destiné à être utilisé dans un procédé pour amorcer une réponse des lymphocytes T cytotoxiques limités au CMH de la classe I avec des peptides exogènes, comprenant les étapes suivantes :
(a) obtention des peptides exogènes et
(b) présentation des peptides exogènes à des cellules présentant des antigènes porteuses de molécules CMH de la classe I en présence d'une quantité de β₂-microglobuline libre, et mise en culture des cellules ainsi préparées en présence d'une population native de lymphocytes T, dans laquelle la β₂-microglobuline est présente en une quantité suffisante pour stimuler une réponse immune et à un niveau supérieur à celui normalement présent sous forme de β₂-microglobuline libre dans le sérum de mammifère.

8. Utilisation d'une β₂-microglobuline selon la revendication 7, dans laquelle la β₂-microglobuline est choisie dans le groupe composé par la β₂-microglobuline humaine purifiée, la β₂-microglobuline murine purifiée, la β₂-microglobuline bovine purifiée et la β₂-microglobuline équine purifiée, et leurs mélanges.

9. Utilisation d'une β₂-microglobuline selon la revendication 8, dans laquelle les peptides exogènes sont présentés à des cellules présentant des antigènes porteuses de molécules CMH de la classe I en présence d'une quantité élevée de β₂-microglobuline libre in vivo.

10. Utilisation d'une β₂-microglobuline pour la préparation d'un agent thérapeutique destiné à être utilisé dans un procédé utilisant une β₂-microglobuline purifiée à titre d'adjuvant pour un peptide vaccin synthétique, comprenant les étapes suivantes : obtention de la β₂-microglobuline purifiée et présentation de polypeptides synthétiques comprenant des épitopes dérivés d'agents pathogènes aux cellules immunes d'un mammifère-hôte en présence de niveaux élevés de β₂-microglobuline libre, dans laquelle la β₂-microglobuline est présente en une quantité suffisante pour stimuler une réponse immune et à un niveau supérieur à celui normalement présent sous forme de β₂-microglobuline libre dans le sérum de mammifère.

11. Utilisation d'une β₂-microglobuline selon la revendication 10, dans laquelle les peptides synthétiques et la β₂-microglobuline libre sont injectés simultanément dans le mammifère-hôte.

12. Utilisation d'une β₂-microglobuline selon la revendication 10, dans laquelle les peptides synthétiques et la β₂-microglobuline libre sont injectés en séquence dans le mammifère-hôte.

13. Utilisation d'une β₂-microglobuline pour la préparation d'un agent thérapeutique destiné à être utilisé dans un procédé pour induire une réponse de la part de lymphocytes T cytotoxiques limités au CMH de la classe I à un immunogène associé à un agent pathogène chez un mammifère-hôte, comprenant les étapes suivantes :
(a) obtention d'un immunogène associé à un agent pathogène et
(b) exposition d'une population native de lymphocytes T à l'immunogène associé à un agent pathogène en présence de β₂-microglobuline libre, ladite β₂-microglobuline étant présente en une concentration supérieure à la concentration de β₂-microglobuline normalement présente dans le sérum de mammifère et suffisante pour induire une réponse immune.

14. Utilisation d'une β₂-microglobuline selon la revendication 13, dans laquelle la β₂-microglobuline est choisie dans le groupe composé par la β₂-microglobuline humaine purifiée, la β₂-microglobuline murine purifiée, la β₂-microglobuline bovine purifiée et la β₂-microglobuline équine purifiée, et leurs mélanges.

15. Utilisation d'une β₂-microglobuline selon la revendication 14, dans laquelle la β₂-microglobuline est une β₂-microglobuline humaine purifiée.

16. Utilisation d'une β₂-microglobuline selon l'une quelconque des revendications 13 à 15, dans laquelle la β₂-microglobuline libre est introduite dans le mammifère-hôte en une quantité qui est d'environ 2 à environ 10 fois supérieure à celle normalement présente dans le sérum dudit mammifère-hôte.
